# EUROPEAN PATENT SPECIFICATION

(11) **EP 2 945 738 B1**
(45) Date of publication and mention of the grant of the patent: **03.06.2020**
(21) Application number: 14701280.1
(22) Date of filing: 14.01.2014
(51) Int. Cl.: B01J 37/02, B01J 37/08, B01J 38/00, B01J 21/12, B01J 21/20, B01J 23/00, B01J 23/34, B01J 23/42, B01J 23/58, B01J 23/62, B01J 23/656, B01J 27/10, B01J 27/13, B01J 27/135, B01J 27/138

(54) **DEHYDROGENATION MANGANESE-CONTAINING CATALYST, ITS USE AND METHOD OF PREPARATION**
MANGANHALTIGER DEHYDRIERUNGSKATALYSATOR, DESSEN VERWENDUNG UND VERFAHREN ZUR HERSTELLUNG
CATALYSEUR DE DÉSHYDROGÉNATION CONTENANT DU MANGANÈSE, SON UTILISATION ET SON PROCÉDÉ DE PRÉPARATION

(30) Priority: 16.01.2013 US 201313742439
(43) Date of publication of application: 25.11.2015
(73) Proprietor: Saudi Basic Industries Corporation, 11422 Riyadh (SA)
(72) Inventor: KAUFFMAN, James W., Sugar Land, Texas 77478 (US); HOOKS, Patricia A., Sugar Land, Texas 77478 (US); NAIR, Balamurali, Sugar Land, Texas 77478 (US)
(74) Representative: Balder IP Law, S.L.
(86) International application number: PCT/US2014/011505
(87) International publication number: WO 2014/113395

(56) References cited:
- EP-A2- 0 186 399
- KR-A- 20120 077 688
- US-A- 4 353 815
- US-A- 4 401 557
- US-A1- 2004 259 727
- US-A1- 2011 263 416

## Description

### FIELD OF THE INVENTION

The present invention relates to catalysts, their preparation and use in the conversion of hydrocarbons by dehydrogenation.

### BACKGROUND

Dehydrogenation reactions are endothermic reactions and require the input of heat to carry the reaction. At higher temperatures higher conversion can be achieved. Thus, higher temperatures are often desirable. At higher temperatures, however, coking of the catalyst increases. In the dehydrogenation of hydrocarbons, such as the dehydrogenation of propane to propylene, platinum is generally used for the active catalyst component for steam-based dehydrogenation reactions. In such reactions, the primary cause of catalyst deactivation is due to the buildup of coke on the catalyst and catalyst support surface. The buildup of coke results in thermal decomposition of the alkane/alkene and eventually inhibits the dehydrogenation reaction at the platinum surface. Catalysts that have accumulated too much coke may become unusable or must undergo a regeneration process. By providing a low coking dehydrogenation catalysts, higher temperatures may be used that result in higher conversions or that may allow the catalysts to be used for longer periods at lower temperatures, and thus extend the time between regenerations when used. The present invention is therefore directed to providing a low-coking dehydrogenation that provides these benefits.

EP 0186399, the closest prior art, is directed to a method and catalyst for converting propane to propylene, wherein the propane is converted to propylene by dehydrogenation over a catalyst comprising a zeolite, such as ZSM-12, ZSM-35, zeolite Y and mordenite, platinum and optionally an oxide of magnesium and/or manganese.

U.S. Patent No. 4,353,815 is directed to dehydrogenatable hydrocarbons dehydrogenated by contacting them at dehydrogenation conditions with a superactive multimetallic catalytic composite comprising a combination of a catalystically effective amount of a pyrolyzed rhenium carbonyl component with a porous carrier material containing a uniform dispersion of catalystically effective amounts of a platinum group component maintained in the elemental metallic state, and of a manganese component. In an example, the attenuated nonacidic multimetallic catalystic composite is a combination of a pyrolyzed rhenium carbonyl component with a porous carrier material containing catalystically effective amounts of an alkali or alkaline earth component, a manganese component, and a platinum group component.

KR 20120077688 is directed to a metallic catalyst for the dehydrogenation with improved metallic catalyst for the dehydrogenation, specifically, the selectivity of the reaction through the control of the catalyst activation through the assistance metal. In an example, the catalyst includes platinum, tin, germanium, or gallium, the alkali metal or the alkaline earth metal, a halogen element, and an assistance metal.

U.S. Patent Application Publication No. 2011/0263416 is directed to a dehydrogenation catalyst having a macropore size and a high active density of platinum, and facilitates material transfer of reactants and products, delays deactivation due to coke formation, and keeps the initial activity constant after being regenerated. In an example, the catalyst includes platinum, an assistant metal, and a halogen component.

### SUMMARY

A novel catalyst composition useful for the dehydrogenation of hydrocarbon compounds is comprised of components (A) - (G), wherein: (A) is a catalyst substrate; (B) is platinum at a level of 0.2 wt.% to 2wt.%; (C) is at least one of germanium, tin, lead, gallium, indium, and titanium, the total amount of component (C) being at a level of 0.2 wt.% to 5 wt.%; (D) is phosphorus at a level of 1 wt.% to 3 wt.%; (E) is at least one of magnesium, calcium, strontium, barium, radium, and a lanthanide, the total amount of component (E) being at a level of 0.1 wt.% to 5 wt.%; (F) is chloride at a level of 0.1 wt.% to 2 wt.%; and (G) is manganese.

In certain embodiments (G) is manganese at a level of 0.05 wt.% to 5 wt.%.

The catalyst substrate may be an alumina substrate, and in particular embodiments may be a crystalline alumina substrate. In various specific embodiments (C) may be tin. In others, (C) may be tin, and (E) may be calcium.

In still other embodiments, (A) may be an alumina substrate, (B) may be platinum at a level of 0.5 wt.% to 1.5 wt.%, the total amount of component (C) may be at a level of 1 wt.% to 4 wt.%, (D) may be phosphorus at a level of 1 wt.% to 3 wt.%, the total amount of component (E) may be at a level of 1% to 5%, (F) may be chloride at a level of 0.15 wt.% to 1.0 wt.%; and (G) may be manganese at a level of 0.1 wt.% to 2.5 wt.%.

In another application, a method of dehydrogenating hydrocarbons is performed by contacting a hydrocarbon feed with a catalyst within a reactor under hydrocarbon conversion reaction conditions to form hydrocarbon conversion products, the catalyst comprising components (A) - (G), wherein: (A) is a catalyst substrate; (B) is platinum at a level of 0.2 wt.% to 2 wt.%; (C) is at least one of germanium, tin, lead, gallium, indium, and titanium, the total amount of component (C) being at a level of 0.2 wt.% to 5 wt.%; (D) is phosphorus at a level of 1 wt.% to 3 wt.%; (E) is at least one of magnesium, calcium, strontium, barium, radium, and a lanthanide, the total amount of component (E) being at a level of 0.1 wt.% to 5 wt.%; (F) is chloride at a level of 0.1 wt.% to 2 wt.%; and (G) is manganese.

In particular embodiments of the method, the hydrocarbon feed is propane and the hydrocarbon conversion products includes propylene.

Steam may be introduced into the reactor along with the hydrocarbon feed in certain applications. The molar ratio of steam to hydrocarbon feed introduced into the reactor may be from1:1 to 10:1.

In some embodiments, the hydrocarbon conversion reaction is carried out free of oxygen (O₂) gas.

The hydrocarbon conversion reaction may be carried out at a temperature of 500°C to 600 °C. In some applications the hydrocarbon feed is introduced into the reactor at a GHSV of 2100 hr⁻¹ to 4500 hr⁻¹.

In certain embodiments of the method, in the catalyst composition used (G) is manganese at a level of 0.05 wt.% to 5 wt.%. In certain instances of the method, (C) is tin, and (E) is calcium.

In particular embodiments, (A) is an alumina substrate, (B) is platinum at a level of 0.5 wt.% to 1.5 wt.%, the total amount of component (C) is at a level of 1 wt.% to 4 wt.%, (D) is phosphorus at a level of 1 wt.% to 3 wt.%, the total amount of component (E) is at a level of 1% to 5%, (F) is chloride at a level of 0.15 wt.% to 1.0 wt.%, and (G) is manganese at a level of 0.1 wt.% to 2.5 wt.%.

In another aspect of the invention, a method of forming a catalyst composition useful for the dehydrogenation of hydrocarbon compounds is performed by combining the following components: (1) a catalyst substrate; (2) a platinum source; (3) at least one of a germanium source, a tin source, a lead source, a gallium source, an indium source, and a titanium source; (4) a phosphorus source; (5) at least one of a magnesium source, a calcium source, a strontium source, a barium source, a radium source, and a lanthanide source; (6) a chloride source; and (7) a manganese source. The combined materials form a final catalyst composition that comprises components (A) - (G), wherein: (A) is the catalyst substrate; (B) is platinum at a level of 0.2 wt.% to 2 wt.%; (C) is at least one of germanium, tin, lead, gallium, indium, and titanium, the total amount of component (C) being at a level of 0.2 wt.% to 5 wt.%; (D) is phosphorus at a level of 1 wt.% to 3 wt.%; (E) is at least one of magnesium, calcium, strontium, barium, radium, and a lanthanide, the total amount of component (E) being at a level of 0.1 wt.% to 5 wt.%; (F) is chloride at a level of 0.1 wt.% to 2 wt.%; and (G) is manganese.

In certain embodiments of the method of forming the catalyst composition, (A) may be an alumina substrate, (B) may be platinum at a level of 0.5 wt.% to 1.5 wt.%, the total amount of component (C) may be at a level of 1 wt.% to 4 wt.%, (D) may be phosphorus at a level of 1 wt.% to 3 wt.%, the total amount of component (E) may be at a level of 1% to 5%, (F) may be chloride at a level of 0.15 wt.% to 1.0 wt.%, and (G) may be manganese at a level of 0.05 wt.% to 5 wt.%.

### BRIEF DESCRIPTION OF THE DRAWINGS

For a more complete understanding of the present invention, and the advantages thereof, reference is now made to the following descriptions taken in conjunction with the accompanying figures, in which:
FIGURE 1 is a plot of the conversion and selectivity for Catalyst A containing manganese (Mn) and Comparative Catalyst B containing iron (Fe) when used in dehydrogenation of propane to form propylene;
FIGURE 2 is a plot of the conversion and selectivity for Comparative Catalyst C containing zinc (Zn) when used in dehydrogenation of propane to form propylene; and
FIGURE 3 is a plot of the conversion and selectivity for Comparative Catalyst D containing cerium (Ce) when used in dehydrogenation of propane to form propylene.

### DETAILED DESCRIPTION

A useful catalyst composition for the dehydrogenation of hydrocarbon compounds is provided by combining a substrate and various materials that provide and/or enhance the catalytic activity of the catalyst. The substrate may be any substrate capable of incorporating the materials described herein that may be used under dehydrogenation reaction conditions without substantially degrading or affecting the substrate or incorporated materials.

Such substrates may be inorganic oxides, and in most cases, the substrate is an alumina (Al₂O₃) substrate. The alumina substrate may be any form of crystalline alumina material, such as η-alumina, 0-alumina, and γ-alumina materials. Those having a large surface area may be particularly useful. The alumina substrate may be thermally stabilized by calcining prior to or during the formation of the final catalyst. The alumina substrate should have a surface area within the range of 50-150 m²/g and a pore diameter range of 90 to 250 Å (Angstroms) to obtain the desired pore size distribution.

It has been unexpectedly found that manganese (Mn) can be incorporated into this dehydrogenation catalyst to provide improved catalyst properties. Although manganese oxides can activate carbon-hydrogen bonds to dehydrogenate alkanes to alkenes, generally, manganese is not found in commercial dehydrogenation catalysts, particularly for the dehydrogenation of lower alkanes, such as propane, for which the invention has particular application, due to its relatively low activity. Also, manganese oxides can also act as oxidation catalysts. Manganese oxides such as Mn₃O₄ have been found to act as a catalyst for a range of reactions, e.g., the oxidation of methane and carbon monoxide, the decomposition of NO, and the catalytic combustion of organic compounds. In view of its ability to oxidize organics and, the inventors, not intending to be limited by theory, suspect that manganese may provide a larger oxygen sink compared to other elements because it can attain a higher oxidation state of 4⁺ to produce MnO₂. This is suspected to facilitate the oxidation and removal of organic coke from the surface of the catalyst and increase catalyst life or lengthen periods between regeneration of the catalyst.

Although the manganese may be present in any amount, it certain embodiments it is present in the catalyst composition at a level of 0.05% to 5% by weight of the catalyst. In certain applications, it is preferably present in the catalyst composition at a level from 0.1% to 2.5% by weight the catalyst. In particular embodiments, the manganese may be present at a level of 0.1% to 1.0% by weight of the catalyst composition, and still more particularly from 0.2% to 0.3% by weight of the catalyst composition. By way of clarification, unless stated otherwise or is otherwise apparent from its context, all percentage amounts are based on weight percentages of the catalyst composition (the substrate together with the added metal components forming the active catalyst without any optional binder materials). Non-limiting examples of suitable sources of manganese include manganese oxides, as well as manganese nitrates, hydroxides, halides, carbonates, acetates, etc. Examples of manganese source are also described in U.S. Patent No. 4,547,618. Typically the manganese source is a manganese oxide or manganese carbonate, and is usually a salt that can be dissolved in water. These materials are applied to or otherwise combined with the catalyst substrate. They are typically dissolved in aqueous fluids that are then used to treat the substrate, such as through impregnation and incipient wetness.

It should be understood that with respect to any concentration or amount range listed or described herein as being useful, suitable, or the like, it is intended to include every concentration or amount within the range, including the end points, and is to be considered as having been specifically stated. For example, "a range of 1 to 10" is to be read as indicating each and every possible number along the continuum between 1 and 10. Thus, even if specific data points within the range, or even no data points within the range, are explicitly identified or refer to only a specific few, it is to be understood that the inventors appreciate and understand that any and all data points within the range are to be considered to have been specified, and that the inventors are in possession of the entire range and all points within the range.

Phosphorus is also combined with the substrate in forming the catalyst composition. The phosphorus may be present in the catalyst composition at a level of 1% to 3% by weight of the catalyst composition. In particular embodiments, the phosphorus is present at a level of 1.1% to 1.5% by weight of the catalyst composition. Examples of suitable sources of phosphorus used in treating the substrate in preparing the catalyst may include, but are not limited to, phosphonic, phosphinous, phosphorus and phosphoric acids, salts and esters of such acids and phosphorous halides. In particular, phosphoric acid (H₃PO₄) and ammonium hydrogen phosphate ((NH₄)₂HPO₄) may be used as the phosphorus source.

The catalyst composition also includes platinum. This material may be combined with the substrate at a level of 0.2% to 2% by weight of the catalyst. In particular embodiments, this component may be present into the catalyst composition at a level of 0.5% to 1.5% by weight of the catalyst composition, and still more particularly 0.7% to 1.2% by weight of the catalyst composition. Non-limiting examples of suitable sources of platinum that may be used in treating the catalyst substrate include chloroplatinic acid, platinous chloride and compounds containing the platinum ammine complex.

Various other materials may be combined with the substrate to form the catalyst composition and may include those described in U.S. Patent No. 6,414,209. Such materials may include at least one element from the group consisting of germanium, tin, lead, gallium, indium, and titanium. These materials may be present in the catalyst composition so that their combined or total amount is at a level of 0.2% to 5% by weight of the catalyst. In other words, if only one of these elements is used, that element may be present at a level anywhere within that range. If more than one of these elements is used the sum of their amounts will fall within this range. In particular embodiments, these components may be present in the catalyst composition where their total is at a level of 1% to 4% by weight of the catalyst composition, and still more particularly 2% to 3.5% by weight of the catalyst composition. Sources of these materials that may be used in treating the catalyst substrate include their nitrates, hydroxides, halides, carbonates, acetates, and other water soluble salts so that they are present in the final catalyst. In particular embodiments tin is used in the catalyst.

The catalyst may further include at least one element from the group consisting of magnesium, calcium, strontium, barium, radium, and a lanthanide. These materials may be present in the catalyst composition so that their combined or total amount is at a level of 0.1% to 5% by weight of the catalyst composition. In particular embodiments, these components may be present so that their total amount is at a level of 1% to 3% by weight of the catalyst composition, and still more particularly 1.2% to 2.0% by weight of the catalyst composition. In particular embodiments, calcium is used in the catalyst. Various sources of these materials that may be used in treating the catalyst substrate so that they are present in the final catalyst.

The catalyst composition may also include chloride. The chloride may be present in the catalyst composition at a level of 0.1% to 2% by weight of the catalyst composition. In particular embodiments, the chloride may be present at a level of 0.15% to 1% by weight of the catalyst composition, and still more particularly 0.17% to 0.6% by weight of the catalyst composition. The chloride may be provided by other components or their sources that are combined with or used in treating the catalyst substrate, such as chloride salts of the various components. In other cases, it may be provided by a separate chloride source, such as hydrochloric acid (HCl), which is used to treat the substrate.

In forming the catalyst, the substrate is treated with the sources of the various materials described above. The substrate may be a η-alumina extrudate that can be obtained from a catalyst manufacturer or can be made by preparing an alumina precursor, forming it into the desired catalyst shape and calcining the alumina precursor to give the final substrate. The substrate or catalyst composition may be configured in various shapes and sizes. In one example, the shape and size may be cylindrical in shape with a 1/8" diameter that may vary in length, such as 1/8" or less to up to several inches. In certain applications, the shape and size may be spherical or tablet-shaped or configured into other shapes, such as a star shape, with the thickness of the particle being of various thicknesses, which may be greater or less than 1/8" thick (e.g., 1/16" to ½").

The alumina substrate is calcined prior to incorporation of the other components to ensure it has the proper surface area and pore structure and crystalline phase. Once calcined, the alumina substrate is impregnated with the metals solution. The alumina can be dried (e.g., 120°C for 2 hours) before the impregnation if desired.

In preparing the catalyst composition, a treatment solution may be made by adding the metal salts, phosphoric acid, chloride source (e.g., HC1) and water together. The treatment solution may then be added all at once to the alumina substrate, which is then mixed until the solution is adequately absorbed into the alumina substrate. The wet alumina substrate may then be dried prior to calcining (e.g., 120°C in air for one or more hours). The dried catalyst particle may then be calcined in air. The calcining may include ramping the temperature in various stages. An example of a typical calcination profile includes increasing the catalyst bed temperature at 5°C/minute to 100°C and soaking in air for 5 hours; increasing the catalyst bed temperature at 5°C/minute to 150°C and soaking in air for 5 hours; increasing the catalyst bed temperature at 5°C/minute to 340°C; increasing the catalyst bed temperature at 1°C/minute to 350°C and soaking in air for 2 hours; increasing the catalyst bed temperature at 10°C/minute to 540°C; increasing the catalyst bed temperature at 1°C/minute to 550°C and soaking in air for 2 hours. Once calcined the catalyst is cooled to room temperature.

The formed catalyst composition may be used hydrocarbon conversion reactions, particularly in dehydrogenation reactions. In particular, the catalyst composition may be used in the dehydrogenation of those paraffins or alkane hydrocarbons of C₂ to C₂₀, more particularly, those hydrocarbon of C₂ to C₅, and still more particularly those from C₃ to C₄. The catalyst composition has particular application in the conversion of propane to propylene.

In use, the catalyst may be used in a reactor and contacted with a hydrocarbon feed that is introduced in the reactor under hydrocarbon conversion conditions to form hydrocarbon conversion products. The hydrocarbon feed may be a paraffin or alkane hydrocarbon feed and the conversion conditions may be those dehydrogenation reaction conditions useful to form dehydrogenated hydrocarbon products, such as a propane feed that is dehydrogenated to form propylene.

A steam or water co-feed is typically used in the reaction with the hydrocarbon feed. The steam or water may act as a carrier gas to facilitate introduction of the hydrocarbon into the reactor. The purpose of using steam is to carry heat into the reactor since the dehydrogenation is an endothermic reaction and to minimize coke formation. Steam is known to at least partially remove or inhibit coke formation on the catalyst. The steam also serves to dilute the hydrocarbon feed so the catalyst is not quickly coked and the reactor is not cooled too much due to the endothermic dehydrogenation reaction. Steam also serves as a diluent that shifts the equilibrium conversion to higher values. In certain applications, the hydrocarbon/H₂O molar feed ratio may range from 1:1 to 10:1, more particularly from 1:2 to 1:6. A hydrocarbon/water molar feed of 1:3 to 1:5 has been found particularly useful for the dehydrogenation of propane.

The dehydrogenation reaction may be a non-oxidative dehydrogenation reaction wherein the reaction is carried out in an oxygen-free (i.e. no oxygen gas or O₂) environment. Furthermore, the reaction may be carried out without any hydrogen gas (H₂) co-feed, as is used in some dehydrogenation reactions. An diluents, which may be an inert diluents such as helium, may also be used in the reaction.

The feed streams may be preheated and introduced into the reactor at temperatures that may range from 200° C to 300 °C. The hydrocarbon, steam and diluent feed may be introduced into the reactor at a GHSV of 2100 hr⁻¹ to 4500 hr⁻¹, more particularly from 3000 hr⁻¹ to 3500 hr⁻¹.

Because the dehydrogenation reaction is endothermic, heat input is typically required to maintain the reaction. The dehydrogenation reaction may be carried out in a tube-type fixed bed reactor that is provided with a heat source to maintain suitable reaction temperatures. Other suitable reactors may be used however. The reaction temperatures are typically maintained at from 525 °C to 610 °C, more particularly from 545 °C to 595 °C.

The catalyst composition of the invention does not tend to coke as readily as existing dehydrogenation catalyst compositions and has been found to provide high conversion and selectivity when used in dehydrogenation reactions. This produces a lower alkane recycle and a higher reaction throughput. The catalyst composition has been found to be particularly useful in providing a high conversion and selectivity in propane dehydrogenation. The lower-coking catalyst also allows the reactor to be run for longer periods of time during the dehydrogenation cycle between any necessary regenerations. This extends the life of the catalyst and reduces overall catalyst and product costs.

The following examples better serve to illustrate the invention.

### EXAMPLES

### EXAMPLE 1

Two catalysts compositions were prepared for use in the dehydrogenation of propane. One catalyst composition was prepared using manganese (Catalyst A) in accordance with the invention. The other catalyst composition (Comparative Catalysts B) utilized iron. The catalyst compositions were each prepared using a η-alumina support. The catalyst compositions were each treated with various compounds in a treatment solution as set forth for Catalyst A and Comparative Catalyst B in Table 1 below to provide the various catalyst components. The substrate was an η-alumina extrudate. The substrate in this case was formed as cylindrical extrudate having a 1/8" diameter.

| Table 1 | | | | |
|---|---|---|---|---|
| Catalyst Component | Catalyst A | | Comparative Catalyst B | |
| | g | ml | g | ml |
| HCl (Conc.) | - | 0.2 | - | 0.2 |
| Water | 3.95 | 3.95 | 3.95 | 3.95 |
| MnCO₃ (dissolved) | 0.08 | - | None | None |
| Fe(NO₃)₃·9H₂O | None | None | 1.42 | - |
| H₃PO₄ | 0.84 | 0.5 | 0.84 | 0.5 |
| SnCl₂·2H₂O | 0.62 | - | 0.62 | - |
| Ca((NO₃)₂·4H₂O | 1.56 | - | 1.56 | - |
| H₂PtCl₆, aqueous at 20wt% Pt | - | 0.5 | - | 0.5 |
| η-alumina | 14 | - | 14 | - |

The alumina substrate was calcined and then impregnated with the treatment solutions prepared beforehand. The alumina was dried at about 120°C for 2 hours before the impregnation. The treatment solution was made by adding the metal salts, phosphoric acid, hydrochloric acid and water together. The treatment solution was then added all at once to the alumina substrate, which was shaken in a container for several minutes until the solution was absorbed into the alumina substrate. The wet alumina substrate was then dried in air at about 120°C for one or more hours to ensure all of the moisture was evaporated.

The dried catalyst particle was then calcined in air. A typical calcination profile included increasing the catalyst bed temperature at 5°C/minute to 100°C and soaking in air for 5 hours; increasing the catalyst bed temperature at 5°C/minute to 150°C and soaking in air for 5 hours; increasing the catalyst bed temperature at 5°C/minute to 340°C; increasing the catalyst bed temperature at 1°C/minute to 350°C and soaking in air for 2 hours; increasing the catalyst bed temperature at 10°C/minute to 540°C; increasing the catalyst bed temperature at 1°C/minute to 550°C and soaking in air for 2 hours. Once calcined the catalyst is cooled to room temperature.

Table 2 shows the percentages of each of the components provided from the treatment.

| Table 2 | | |
|---|---|---|
| Catalyst Component | Catalyst A | Comparative Catalyst B |
| | Wt.% | Wt.% |
| Cl | ∼0.1-0.4 | 0.27 |
| Mn | 0.28 | None |
| Fe | None | 1.31 |
| Zn | None | None |
| Ce | None | None |
| P | 1.21 | 1.05 |
| Sn | 2.96 | 1.42 |
| Ca | 1.54 | 1.49 |
| Pt | 0.84 | 0.77 |
| η-alumina | 92.0 | 91.4 |

### EXAMPLE 2

Catalyst A and Comparative Catalyst B were each used in propane dehydrogenation reactions to produce propylene at substantially the same reaction conditions. The catalyst samples were tested in a microreactor by blending the individual feed gases together, preheating the gas mixture and then passing the mixture over a catalyst sample in a reactor tube. The feed gases consisted of propane, steam, with and without a helium diluent, generally at flow rates of 5 sccm, 20 sccm, and 2 sccm when helium was used. Each gas including the propane and diluent gas such as helium was high purity of +99% and were metered into the reactor with gas mass flow meters. The steam was introduced into the reactor by metering water using an ISCO brand pump into a preheater at 200°C to convert it into steam. The steam was them fed into a tube mixer with the propane and helium. The microreactor was a 316 stainless steel fixed bed reactor coated internally with a silica vapor phase coating to minimize dehydrogenation and side reactions that may occur on the reactor wall surface. The reactor had a 0.5 inch inner diameter with a catalyst charge of 0.5 g. The hydrocarbon/H₂O molar feed ratio ranged from about 1:3-1:4.

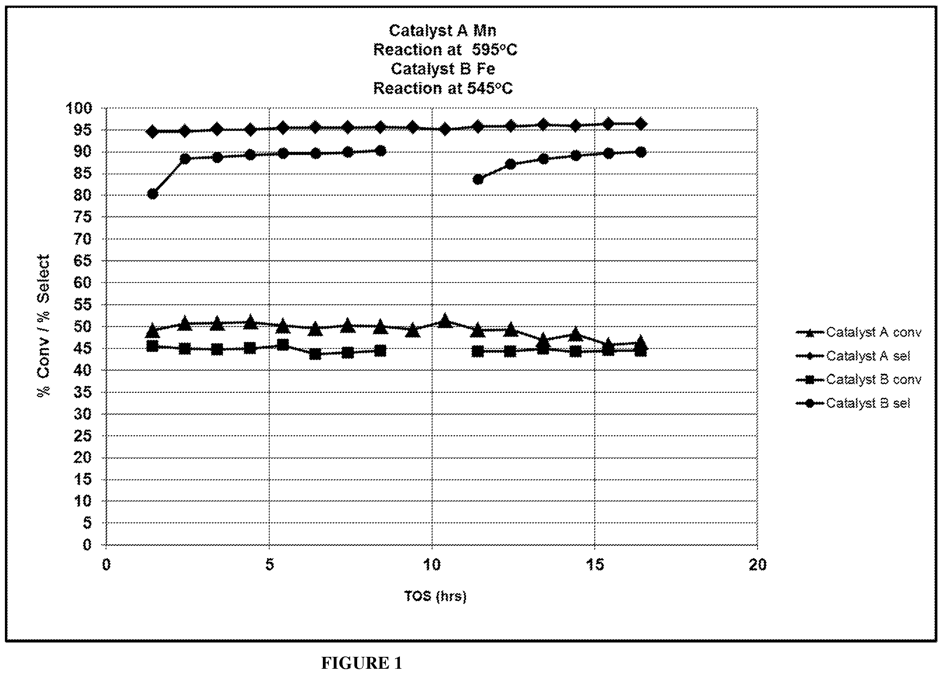

The feed streams were preheated to a temperature of 200 °C to 300 °C. The gases were introduced at about a 1.2 WHSV hr⁻¹.Because the dehydrogenation reaction is endothermic, heat was input into the reactor to maintain the reaction. The reactor temperature was generally maintained at a temperature of 595 °C during the reactions. The effluent gases were then analyzed using a Varian gas TCD chromatograph. The results are presented in Figure 1.

As can be seen from Figure 1, Catalyst A had better selectivity and lower coking. This is observed in that Catalyst A had higher selectivity. A lower selectivity indicates higher coking, as more of the alkane is being decomposed to coke as the selectivity decreases. It is apparent from the selectivity curves of Figure 1 that Catalyst A containing manganese gave higher selectivity and therefore lower coking, even at higher conversion compared to the iron-containing catalyst, Comparative Catalyst B. This indicates better performance when manganese is utilized. Furthermore, Catalyst A did not include any regeneration. Comparative Catalyst B included a regeneration after eight hours on stream, as indicated by the interrupted plot of Figure 1. Even without a regeneration, the manganese catalyst conversion was still higher.

### COMPARATIVE EXAMPLE 3

A comparative catalyst composition (Comparative Catalyst C) utilizing zinc was prepared in a similar manner to that of Example 1. The catalyst composition was prepared using a η-alumina support. The catalyst composition was treated with a treatment solution containing the various compounds as set forth in Table 3 below to provide the various catalyst components, which are compared to those of Catalyst A, previously discussed. Table 4 shows the percentages of each of the components provided from the treatment.

| Table 3 | | | | |
|---|---|---|---|---|
| Catalyst Component | Catalyst A | | Comparative Catalyst C | |
| | g | ml | g | ml |
| HCl (Conc.) | - | 0.2 | - | 0.2 |
| Water | 3.95 | 3.95 | 2.021 | 2.021 |
| MnCO₃ (dissolved) | 0.08 | - | None | None |
| Zn acetate | None | None | 0.128 | - |
| H₃PO₄ | 0.84 | 0.5 | 0.84 | 0.5 |
| SnCl₂·2H₂O | 0.62 | - | 0.62 | - |
| Ca((NO₃)₂·4H₂O | 1.56 | - | 1.56 | - |
| H₂PtCl₆, aqueous at 20wt% Pt | - | 0.5 | - | 0.5 |
| η-alumina | 14 | - | 14 | - |

| Table 4 | | |
|---|---|---|
| Catalyst Component | Catalyst A | Comparative Catalyst C |
| | Wt.% | Wt.% |
| Cl | ∼0.1-0.4 | ∼0.1-0.4 |
| Mn | 0.28 | None |
| Fe | None | None |
| Zn | None | 0.2 |
| Ce | None | None |
| P | 1.21 | 0.9 |
| Sn | 2.96 | 2.1 |
| Ca | 1.54 | 1.1 |
| Pt | 0.84 | 0.7 |
| η-alumina | 92.0 | 92 |

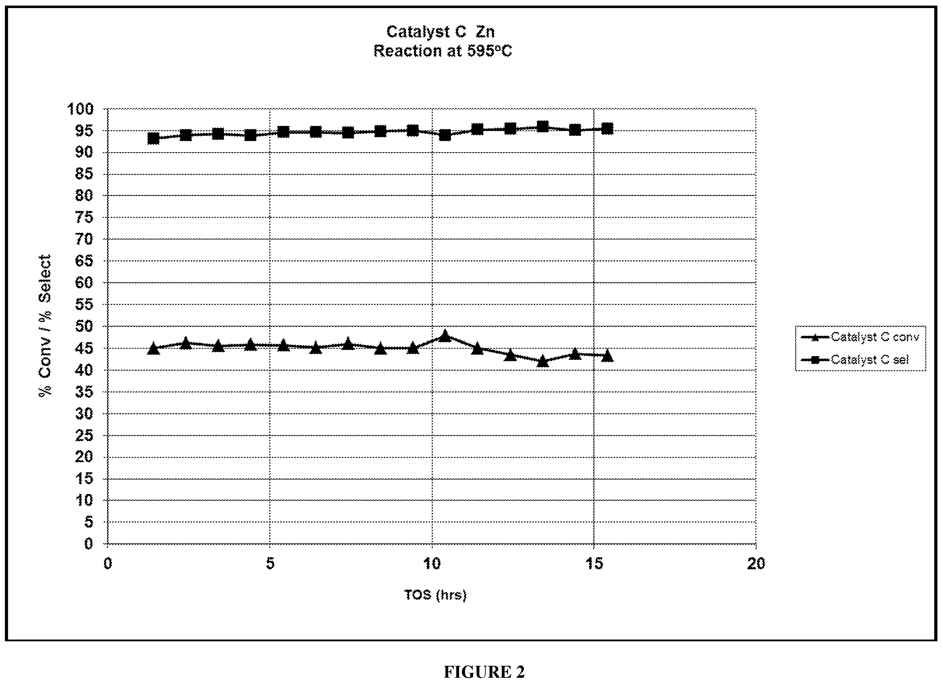

Comparative Catalyst C was used in propane dehydrogenation reactions to produce propylene at substantially the same reaction conditions as those described for Example 2. The run data for the zinc catalyst composition (Comparative Catalyst C) is shown for conversion and selectivity at 595°C in Figure 2. When compared against the Mn containing catalyst composition (Catalyst A) in Figure 1, the activity of the Zn catalyst composition is not as high, as indicated by a lower conversion at about 45% compared to about 50% for the Mn catalyst.

It is apparent that the Zn catalyst composition (Comparative Catalyst C) is not as active as the Mn catalyst composition (Catalyst A) under the same reaction conditions, showing only a 45% conversion of propane to propylene compared to about 50% for Catalyst A.

### COMPARATIVE EXAMPLE 4

A comparative catalyst composition (Comparative Catalyst D) utilizing cerium was prepared in a similar manner to that of Example 1. The catalyst composition was prepared using a η-alumina support. The catalyst composition was treated with a treatment solution containing the various compounds as set forth in Table 5 below to provide the various catalyst components, which are compared to those of Catalyst A, previously discussed. Table 6 below shows the percentages of each of the components provided from the treatment.

| Table 5 | | | | |
|---|---|---|---|---|
| Catalyst Component | Catalyst A | | Comparative Catalyst D | |
| | g | ml | g | ml |
| HCl (Conc.) | - | 0.2 | - | 0.2 |
| Water | 3.95 | 3.95 | 3.95 | 3.95 |
| MnCO₃ (dissolved) | 0.08 | - | None | None |
| Ce(NO3)3*6H2O | None | None | 0.32 | - |
| H₃PO₄ | 0.84 | 0.5 | 0.84 | 0.5 |
| SnCl₂·2H₂O | 0.62 | - | 0.62 | - |
| Ca((NO₃)₂·4H₂O | 1.56 | - | 1.56 | - |
| H₂PtCl₆, aqueous at 20wt% Pt | - | 0.5 | - | 0.5 |
| η-alumina | 14 | - | 14.1 | - |

| Table 6 | | |
|---|---|---|
| Catalyst Component | Catalyst A | Comparative Catalyst D |
| | Wt.% | Wt.% |
| Cl | ∼0.1-0.4 | ∼0.1-0.4 |
| Mn | 0.28 | None |
| Fe | None | None |
| Zn | None | None |
| Ce | None | 0.54 |
| P | 1.21 | 1.23 |
| Sn | 2.96 | 3.15 |
| Ca | 1.54 | 1.57 |
| Pt | 0.84 | 0.89 |
| η-alumina | 92.0 | 91.5 |

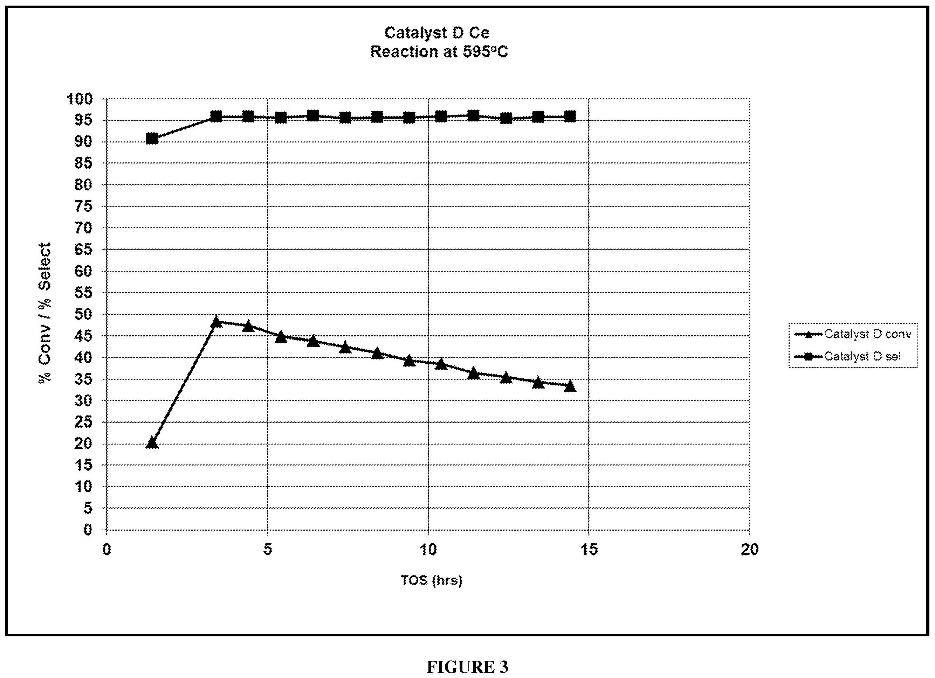

Comparative Catalyst D was used in propane dehydrogenation reactions to produce propylene at substantially the same reaction conditions as those described for Example 2. The run data for the cerium catalyst composition (Comparative Catalyst D) is shown for conversion and selectivity at 595°C in Figure 3. It is apparent that the Ce catalyst composition (Comparative Catalyst D) is not as active as the Mn catalyst composition (Catalyst A) under the same reaction conditions, showing only a 47% conversion of propane to propylene compared to about 50% for the Mn catalyst composition (Catalyst A). Comparative Catalyst D also showed a faster decrease in activity, indicating that it coked faster than the Mn catalyst (Catalyst A).

While the invention has been shown in only some of its forms, it should be apparent to those skilled in the art that it is not so limited, but is susceptible to various changes and modifications without departing from the scope of the invention. Accordingly, it is appropriate that the appended claims be construed broadly and in a manner consistent with the scope of the invention.

## Claims

1. A catalyst composition useful for the dehydrogenation of hydrocarbon compounds comprising:
components (A) - (G), wherein:
(A) is a catalyst substrate;
(B) is platinum at a level of 0.2 wt.% to 2wt.% based on the weight of the catalyst composition;
(C) is at least one of germanium, tin, lead, gallium, indium, and titanium, the total amount of component (C) being at a level of 0.2 wt.% to 5 wt.% based on the weight of the catalyst composition;
(D) is phosphorus at a level of 1 wt.% to 3 wt.% based on the weight of the catalyst composition;
(E) is at least one of magnesium, calcium, strontium, barium, radium, and a lanthanide, the total amount of component (E) being at a level of 0.1 wt.% to 5 wt.% based on the weight of the catalyst composition;
(F) is chloride at a level of 0.1 wt.% to 2 wt.% based on the weight of the catalyst composition; and
(G) is manganese.

2. The catalyst composition of Claim 1, wherein (G) is manganese at a level of 0.05 wt.% to 5 wt.% based on the weight of the catalyst composition.

3. The catalyst composition of any of Claims 1 - 2, wherein (G) is manganese at a level of 0.1 wt.% to 2.5 wt.% .% based on the weight of the catalyst composition.

4. The catalyst composition of any of Claims 1 - 3, wherein (A) is an alumina substrate; preferably wherein (A) is a crystalline alumina substrate.

5. The catalyst composition of any of Claims 1 - 4, wherein (C) is tin.

6. The catalyst composition of any of Claims 1 - 5, wherein (E) is calcium.

7. The catalyst composition of any of Claims 1 - 6, wherein:
(B) is platinum at a level of 0.5 wt.% to 1.5 wt.% .% based on the weight of the catalyst composition;
the total amount of component (C) is at a level of 1 wt.% to 4 wt.% .% based on the weight of the catalyst composition;
(D) is phosphorus at a level of 1 wt.% to 3 wt.% .% based on the weight of the catalyst composition;
the total amount of component (E) is at a level of 1% to 5%.% based on the weight of the catalyst composition; and
(F) is chloride at a level of 0.15 wt.% to 1.0 wt.% .% based on the weight of the catalyst composition.

8. A method of converting hydrocarbons by dehydrogenation comprising contacting a hydrocarbon feed with the catalyst composition of any of Claims 1 - 7 within a reactor under dehydrogenation conversion reaction conditions to form dehydrogenated hydrocarbon conversion products.

9. The method of Claim 8, wherein: the hydrocarbon feed is a paraffin hydrocarbon feed; preferably, wherein the hydrocarbon feed is propane and the hydrocarbon conversion products include propylene.

10. The method of any of Claims 8-9, wherein steam is introduced into the reactor along with the hydrocarbon feed.

11. The method of Claim 10, wherein molar ratio of steam to hydrocarbon feed introduced into the reactor is from1 : 1 to 10:1.

12. The method of any of Claims 8 - 11, wherein the hydrocarbon conversion reaction is carried out free of oxygen (O₂) gas.

13. The method of any of Claims 8 - 12, wherein the hydrocarbon conversion reaction is carried out at a temperature of 500°C to 600°C.

14. The method of any of Claims 8 - 13, wherein the hydrocarbon feed is introduced into the reactor at a GHSV of 2100 hr⁻¹ to 4500 hr⁻¹.

15. A method of forming the catalyst composition of any of Claims 1 - 7 useful for the dehydrogenation of hydrocarbon compounds, the method comprising:
combining the following components:
(1) a catalyst substrate;
(2) a platinum source;
(3) at least one of a germanium source, a tin source, a lead source, a gallium source, an indium source, and a titanium source;
(4) a phosphorus source;
(5) at least one of a magnesium source, a calcium source, a strontium source, a barium source, a radium source, and a lanthanide source;
(6) a chloride source; and
(7) a manganese source;
to form the catalyst composition.

## Patentansprüche

1. Eine für die Dehydrierung von Kohlenwasserstoffverbindungen geeignete Katalysatorzusammensetzung bestehend aus:
Komponenten (A) - (G), wobei:
(A) ein Katalysatorsubstrat ist;
(B) Platin in einer Menge von 0,2 Gew.-% bis 2 Gew.-%, bezogen auf das Gewicht der Katalysatorzusammensetzung, ist;
(C) zumindest eines der Elemente Germanium, Zinn, Blei, Gallium, Indium und Titan ist, wobei die Gesamtmenge der Komponente (C) 0,2 Gew.-% bis 5 Gew.-%, bezogen auf das Gewicht der Katalysatorzusammensetzung, beträgt;
(D) Phosphor in einer Menge von 1 Gew.-% bis 3 Gew.-%, bezogen auf das Gewicht der Katalysatorzusammensetzung, ist;
(E) zumindest eines der Elemente Magnesium, Kalzium, Strontium, Barium, Radium und ein Lanthanid ist, wobei die Gesamtmenge der Komponente (E) 0,1 Gew.-% bis 5 Gew.-%, bezogen auf das Gewicht der Katalysatorzusammensetzung, beträgt;
(F) Chlorid in einer Menge von 0,1 bis 2 Gew.-%, bezogen auf das Gewicht der Katalysatorzusammensetzung, ist; und
(G) Mangan ist.

2. Katalysatorzusammensetzung nach Anspruch 1, wobei (G) Mangan in einer Menge von 0,05 Gew.-% bis 5 Gew.-%, bezogen auf das Gewicht der Katalysatorzusammensetzung, ist.

3. Die Katalysatorzusammensetzung nach einem der Ansprüche 1 bis 2, wobei (G) Mangan in einer Menge von 0,1 Gew.-% bis 2,5 Gew.-%, bezogen auf das Gewicht der Katalysatorzusammensetzung, ist.

4. Die Katalysatorzusammensetzung nach einem der Ansprüche 1 bis 3, wobei (A) ein Aluminiumoxidsubstrat ist; vorzugsweise wobei (A) ein kristallines Aluminiumoxidsubstrat ist.

5. Die Katalysatorzusammensetzung nach einem der Ansprüche 1-4, wobei (C) Zinn ist.

6. Die Katalysatorzusammensetzung nach einem der Ansprüche 1 bis 5, wobei (E) Calcium ist.

7. Die Katalysatorzusammensetzung nach einem der Ansprüche 1 bis 6, wobei:
(B) Platin in einer Menge von 0,5 Gew.-% bis 1,5 Gew.-%, bezogen auf das Gewicht der Katalysatorzusammensetzung, ist; die Gesamtmenge der Komponente (C) bei einem Niveau von 1 Gew.-% bis 4 Gew.-%, basierend auf der Gewicht der Katalysatorzusammensetzung, liegt;
(D) Phosphor in einer Menge von 1 Gew.-% bis 3 Gew.-%, bezogen auf das Gewicht der Katalysatorzusammensetzung, ist;
die Gesamtmenge der Komponente (E) bei 1% bis 5%, bezogen auf das Gewicht der der Katalysatorzusammensetzung, liegt; und
(F) Chlorid in einer Menge von 0,15 Gew.-% bis 1,0 Gew.-%, bezogen auf das Gewicht der Katalysatorzusammensetzung, ist.

8. Verfahren zur Umwandlung von Kohlenwasserstoffen durch Dehydrierung umfassend den Kontakt einer Kohlenwasserstoffbeschickung mit der Katalysatorzusammensetzung nach einem der Ansprüche 1 - 7 in einem Reaktor unter Dehydrierungsumwandlungreaktionsbedingungen zur Bildung von dehydrierten Kohlenwasserstoff-Umwandlungsprodukten.

9. Verfahren nach Anspruch 8, wobei: die Kohlenwasserstoffbeschickung eine Paraffinkohlenwasserstoffbeschickung ist; vorzugsweise wobei die Kohlenwasserstoffbeschickung Propan ist und die Kohlenwasserstoffumwandlungsprodukte Propylen enthalten.

10. Verfahren nach einem der Ansprüche 8-9, bei dem Dampf zusammen mit der Kohlenwasserstoffbeschickung in den Reaktor eingeleitet wird.

11. Verfahren nach Anspruch 10, wobei ein molares Verhältnis von Dampf zu Kohlenwasserstoffbeschickung, die in den Reaktor eingeführt wird, von 1:1 bis 10:1 beträgt.

12. Verfahren nach einem der Ansprüche 8 - 11, bei dem die Kohlenwasserstoff-Umwandlungsreaktion frei von Sauerstoff (O₂)-Gas durchgeführt wird.

13. Verfahren nach einem der Ansprüche 8 - 12, wobei die Kohlenwasserstoff-Umwandlungsreaktion bei einer Temperatur von 500°C bis 600°C durchgeführt wird.

14. Verfahren nach einem der Ansprüche 8 - 13, bei dem die Kohlenwasserstoffbeschickung mit einer GHSV von 2100 h⁻¹ bis 4500 h⁻¹ in den Reaktor eingeführt wird.

15. Verfahren zur Bildung der Katalysatorzusammensetzung nach einem der Ansprüche 1 - 7, die für die Dehydrierung von Kohlenwasserstoffverbindungen geeignet ist, wobei das Verfahren umfasst:
die die folgenden Komponenten kombinieren:
(1) ein Katalysator-Substrat;
(2) eine Platinquelle;
(3) zumindest eine aus einer Germaniumquelle, einer Zinnquelle, einer Bleiquelle, einer Galliumquelle, einer Indiumquelle und einer Titanquelle;
(4) eine Phosphorquelle;
(5) zumindest eine aus einer Magnesiumquelle, einer Kalziumquelle, einer Strontiumquelle, einer Bariumquelle, einer Radiumquelle und einer Lanthanidquelle;
(6) eine Chloridquelle; und
(7) eine Manganquelle;
zur Bildung der Katalysatorzusammensetzung.

## Revendications

1. Composition de catalyseur utile pour la déshydrogénation de composés hydrocarbonés comprenant :
des composants (A) à (G), dans laquelle :
(A) est un substrat de catalyseur ;
(B) est du platine à un taux de 0,2 % en poids à 2 % en poids sur la base du poids de la composition de catalyseur ;
(C) est au moins un élément choisi parmi le germanium, l'étain, le plomb, le gallium, l'indium et le titane, la quantité totale de composant (C) étant à un taux de 0,2 % en poids à 5 % en poids sur la base du poids de la composition de catalyseur ;
(D) est du phosphore à un taux de 1 % en poids à 3 % en poids sur la base du poids de la composition de catalyseur ;
(E) est au moins un élément choisi parmi le magnésium, le calcium, le strontium, le baryum, le radium et un lanthanide, la quantité totale de composant (E) étant à un taux de 0,1 % en poids à 5 % en poids sur la base du poids de la composition de catalyseur ;
(F) est un chlorure à un taux de 0,1 % en poids à 2 % en poids sur la base du poids de la composition de catalyseur ; et
(G) est du manganèse.

2. Composition de catalyseur selon la revendication 1, dans laquelle (G) est du manganèse à un taux de 0,05 % en poids à 5 % en poids sur la base du poids de la composition de catalyseur.

3. Composition de catalyseur selon l'une quelconque des revendications 1 à 2, dans laquelle (G) est du manganèse à un taux de 0,1 % en poids à 2,5 % en poids sur la base du poids de la composition de catalyseur.

4. Composition de catalyseur selon l'une quelconque des revendications 1 à 3, dans laquelle (A) est un substrat d'alumine ; de préférence dans laquelle (A) est un substrat d'alumine cristalline.

5. Composition de catalyseur selon l'une quelconque des revendications 1 à 4, dans laquelle (C) est de l'étain.

6. Composition de catalyseur selon l'une quelconque des revendications 1 à 5, dans laquelle (E) est du calcium.

7. Composition de catalyseur selon l'une quelconque des revendications 1 à 6, dans laquelle :
(B) est du platine à un taux de 0,5 % en poids à 1,5 % en poids sur la base du poids de la composition de catalyseur ;
la quantité totale de composant (C) est à un taux de 1 % en poids à 4 % en poids sur la base du poids de la composition de catalyseur ;
(D) est du phosphore à un taux de 1 % en poids à 3 % en poids sur la base du poids de la composition de catalyseur ;
la quantité totale de composant (E) est à un taux de 1 % à 5 % sur la base du poids de la composition de catalyseur ; et
(F) est un chlorure à un taux de 0,15 % en poids à 1,0 % en poids sur la base du poids de la composition de catalyseur.

8. Procédé de conversion d'hydrocarbures par déshydrogénation comprenant la mise en contact d'une charge d'hydrocarbures avec la composition de catalyseur selon l'une quelconque des revendications 1 à 7 à l'intérieur d'un réacteur dans des conditions de réaction de conversion par déshydrogénation pour former des produits de conversion d'hydrocarbures déshydrogénés.

9. Procédé selon la revendication 8, dans lequel : la charge d'hydrocarbures est une charge d'hydrocarbures de paraffine ; de préférence, dans lequel la charge d'hydrocarbures est du propane et les produits de conversion d'hydrocarbures incluent du propylène.

10. Procédé selon l'une quelconque des revendications 8 à 9, dans lequel de la vapeur est introduite dans le réacteur conjointement avec la charge d'hydrocarbures.

11. Procédé selon la revendication 10, dans lequel le rapport molaire de la vapeur à la charge d'hydrocarbures introduite dans le réacteur est de 1/1 à 10/1.

12. Procédé selon l'une quelconque des revendications 8 à 11, dans lequel la réaction de conversion d'hydrocarbures est réalisée sans gaz d'oxygène (O₂) •

13. Procédé selon l'une quelconque des revendications 8 à 12, dans lequel la réaction de conversion d'hydrocarbures est réalisée à une température de 500 °C à 600 °C.

14. Procédé selon l'une quelconque des revendications 8 à 13, dans lequel la charge d'hydrocarbures est introduite dans le réacteur à une GHSV de 2 100 h⁻¹ à 4 500 h⁻¹.

15. Procédé de formation de la composition de catalyseur selon l'une quelconque des revendications 1 à 7 utile pour la déshydrogénation de composés hydrocarbonés, le procédé comprenant :
la combinaison des composants suivants :
(1) un substrat de catalyseur ;
(2) une source de platine ;
(3) au moins une source choisie parmi une source de germanium, une source d'étain, une source de plomb, une source de gallium, une source d'indium et une source de titane ;
(4) une source de phosphore ;
(5) au moins une source choisie parmi une source de magnésium, une source de calcium, une source de strontium, une source de baryum, une source de radium et une source de lanthanide ;
(6) une source de chlorure ; et
(7) une source de manganèse ;
pour former la composition de catalyseur.
